# EUROPEAN PATENT APPLICATION

(11) **EP 4 154 853 A2**
(43) Date of publication of application: **29.03.2023**
(21) Application number: 22198175.6
(22) Date of filing: 27.09.2022
(51) Int. Cl.: A61F 11/08

(54) **EARPLUGS**

(30) Priority: 27.09.2021 GB 202113776
(71) Applicant: Speedo International Limited, London W1U 3PH (GB)
(72) Inventor: PYBUS, James, London (GB); JOHNSON, Chris, London (GB); ADAMS, Caroline, London (GB)
(74) Representative: Mewburn Ellis LLP

(57) **Abstract**

An earplug (100) comprising a base (105), an elongate stem (102) and one or more flanges (107a, 107b) provided on the stem (102). The elongate stem (102) extends from the base (105) for insertion into a user's ear canal. The stem (102) comprises a proximal portion proximate to the base (105) and a distal portion distal from the base (105). The distal portion extends at an angle to the proximal portion. Also discloses is a pair of earplugs that are releasably engageable with one another.

## Description

### Field of the Disclosure

The present disclosure relates to earplugs, in particular to earplugs for use in sporting activities such as swimming.

### Background

It is often desirable for a wearer partaking in sporting activities to wear earplugs to protect the wearer's ear canal from an ingress of water and/or foreign bodies (e.g. to protect from dirt, sand ingress etc.). For example, swimmers may wear earplugs to prevent water (e.g. seawater or swimming pool water) from entering too deep into a wearer's ear canal as this can cause discomfort for the wearer and even impairment to the wearer's hearing.

In human anatomy, an 'ear canal' refers to a pathway, formed by a hollow cavity in the temporal bone of a human skull, and runs from an 'outer ear' (the external part of the ear) up to the eardrum of the wearer.

Prior art swimming earplugs typically comprise an elongate stem for insertion into the ear canal of the wearer. The stem extends from a base of the earplug and further comprises one or more flanges. When the earplug is inserted into the ear canal the flanges abut against internal walls of the ear canal and collectively form a seal or barrier within the ear canal of the wearer. It is the intention that this seal reduces the chance of water/debris passing into the wearer's ear canal and reaching the eardrum.

These known ear plugs can be a poor fit for some users. A poor fit can be uncomfortable for a wearer. A poor fit can also result in a poor performance of the earplug, as the flanges may be unable to provide an adequate seal between the external environment and the internal walls of the ear canal of the wearer. This is particularly problematic if the wearer is swimming, as it can result in water/debris passing into the wearer's ear canal and reaching the eardrum. This can cause discomfort for the wearer and increases the possibility of hearing impairment.

Earplugs are commonly bought and owned as a pair. However due to their relatively small size and the nature of the sporting activities that the wearer partakes in (e.g. swimming), it is very common for at least one the earplugs to become lost or displaced during use. The problem is compounded if the earplugs are a poor fit for the wearer, as previously discussed.

There is a need to alleviate at least one of the issues described above.

### Summary

In a first aspect, there is provided an earplug comprising:
a base;
an elongate stem extending from the base for insertion into a user's ear canal, the stem comprising a proximal portion proximate to the base and a distal portion distal from the base, the distal portion extending at an angle to the proximal portion; and
one or more flanges provided on the stem.

By providing an elongate stem that includes a distal portion angled relative to a proximal portion (i.e. so as to include a bend therebetween), the elongate stem better matches the internal shape of the user's ear canal and allows the one or more flanges of the earplug to abut against internal walls of the ear canal more intimately. This has a technical effect of improving the seal, formed by the flanges, between the external environment and the internal walls of the user's ear canal and thus improves the performance of the earplug. For an earplug used during swimming, this improved performance includes preventing water/debris passing into the user's ear canal and reaching the eardrum, thus reducing both discomfort for the user and the possibility of hearing impairment. Another advantage is that the earplug fits snugly into the ear canal which improves the overall comfort of the earplug when worn.

Furthermore, unless otherwise stated, references to the shape of the stem and/or flanges herein are references to the shape of those features when the earplug is in a resting/neutral position (i.e. not inserted into a wearer's ear).

The term "base" refers to part of the earplug that remains external to, or outside of, the ear canal when it is use (i.e. when it is being worn), whereas the terms "stem" or "elongate stem" refer to part of the earplug that fits inside the ear canal of the user when it is in use.

Optional features of the invention will now be set out. These are applicable singly or in any combination with any aspect of the invention.

The stem may be curved along at least a portion of its length. The proximal portion may be curved along its length. The distal portion may be curved along its length. A portion of the stem interposed between the proximal and distal portions may be curved along its length. The stem may be curved along its entire length. The radius of curvature of the proximal portion may be less than the radius of curvature of the distal portion.

In some embodiments, the proximal portion may be substantially linear whilst the distal portion is curved. In some embodiments, both the proximal portion and distal portion may be substantially linear but angled relative to one another so as to approximate a curve. In some embodiments the proximal portion and distal portion may be linear and connected by a curved portion.

For the avoidance of doubt, the reference to the stem being curved along at least a portion of its length is a reference to the stem extending along a path and at least a portion of that path being curved (i.e. the stem having a curved axial length).

The proximal portion may be directly connected to (e.g. integral with) the base. The proximal portion may be directly adjacent the base. The distal portion may represent the distalmost portion of the stem.

The base may be enlarged with respect to the stem. That is, the base may have a larger width/diameter than the stem. In some embodiments, the base may comprise opposing major surfaces (having larger areas than any minor surfaces). The major surfaces may be, for example, substantially circular.

The base may define a (reference) base plane. For example, the base plane may be parallel to one or both of the major surfaces. Alternatively or additionally, the base plane may be a plane along which the base extends. For example, the base may have planar (e.g. flattened) shape so as to be thinner along one axis (that is substantially normal to the base plane).

In a reference orientation the base plane may be substantially vertical (extending along an upward/downward axis). In this orientation, a forward/rearward axis may also lie on the base plane and be orthogonal to the upward/downward (i.e. vertical) axis. The forward direction may be in a direction towards the front of a user's head and the rearward direction may be in a direction towards the back of a user's head. An inward/outward axis may extend normally with respect to the base plane. The inward direction may be a direction towards a user's head and the outward direction may be a direction away from a user's head. The reference orientation may be representative of one orientation in which the earplug may be arranged in use. Of course, it should be appreciated that different anatomies between users will mean there may be variation in the actual orientation of the earplug in use.

The proximal portion of the stem may extend inwardly from the base (e.g. the base plane) on an oblique angle (i.e. neither normal nor parallel to the base plane). An inner angle between the proximal portion (e.g. a central line of the proximal portion) and the base plane may be between 90 degrees and 45 degrees, or between 85 degrees and 60 degrees. The inner angle may be between 70 degrees and 75 degrees (e.g. about 74 degrees). The proximal portion may be perpendicular to the base (e.g. base plane).

The proximal portion may extend from the base in an inward and upward in direction. The proximal portion may be angled with respect to the base (e.g. base plane) so as to extend inwardly and upwardly (i.e. so as to extend in a direction that has inward and upward components) in the reference orientation. Thus, when viewed from the front in the reference orientation, the proximal portion may extend on an incline from the base plane. The proximal portion may additionally extend rearwardly (i.e. have a rearward component).

The distal portion may be angled with respect to the base plane so as to extend inwardly and upwardly (i.e. so as to extend in a direction that has inward and upward components) in the reference orientation. Thus, when viewed from the front in the reference orientation, the proximal portion may extend on an incline with respect to the base plane.

The distal portion may be angled with respect to the base plane so as to extend inwardly and rearwardly (i.e. the direction of the distal portion may have a rearward component). Accordingly, the distal portion may extend inwardly, upwardly and rearwardly.

The distal portion may extend inwardly and rearwardly from the proximal portion. The distal portion may be angled rearwardly from the proximal portion (i.e. may be directed more rearwardly than the proximal portion). Thus, when viewed from the above in the reference orientation, an inner angle between the distal portion and the base plane may be smaller than an inner angle between the proximal portion and the base plane.

In some embodiments, the stem (e.g. a central line thereof) may extend within a single plane. The proximal and distal portions (e.g. central lines extending therethrough) may extend within a single plane. That is, the (e.g. curved) path along which the stem extends may be in a single reference plane. However, in some embodiments, the stem (e.g. the distal portion of the stem) may additionally curve or extend upwards (relative to the position of the earplug in use) out of this plane so that the curve/path is in three dimensions.

The stem may consist of the proximal and distal portions. For example, each of the proximal and distal portions may represent approximately half of the length of the stem. The distal portion may be directly connected to the proximal portion (i.e. with no portion provided therebetween). That is, the distal portion may extend from the proximal portion.

The proximal portion of the stem may have a transverse cross-sectional area (perpendicular to the elongation of the stem) that decreases in a direction away from the base (e.g. decreases in a gradual and/or continuous manner). In this way, the stem may have a maximum cross section adjacent the base to as to provide a secure attachment to the base.

The distal portion of the stem may have a substantially unform transverse cross section e.g. with a cross-sectional area substantially equal to the minimum cross-sectional area of the proximal portion.

In some embodiments the transverse cross-sectional area of the stem may decrease in a direction away from the base.

The one or more flanges are disposed on the stem. For example, the flanges may be integrally formed with the stem. In some embodiments, the flanges may be formed of the same material as the stem. The or each flange may be flexible.

In some embodiments, the one or more flanges may consist of two flanges i.e. there may be only two (i.e. and no more than two) flanges on the stem.

In some embodiments, the earplug comprises a distal flange disposed (e.g. integrally formed) on the distal portion of the elongate stem. In some embodiments, the earplug comprises a proximal flange disposed (e.g. integrally formed) on the proximal portion of the elongate stem. The proximal flange may be disposed centrally between the distal flange and the base.

The proximal flange may be larger than the distal flange. For example, the proximal flange may have a larger maximum diameter than the maximum diameter of the distal flange.

This allows the distal flange to abut against the internal walls of the user's ear canal at its narrower (deeper) section, whereas the larger proximal flange can abut against the internal walls of the ear canal at the wider (shallower) section. In this way, the different sized maximum diameters of the two flanges match the ear canal at its widest and narrowest sections, which further improves the overall comfort and performance of the earplug when in use.

Owing to the angled proximal and distal portions, the planes in which the maximum diameters of the flanges reside will not be parallel. The planes will each be angled differently relative to the base e.g. relative to the major surface of the base from which the stem extends. For example, the maximum diameter plane of the proximal flange may form an angle with the maximum diameter plane of the distal plane (e.g. viewed from above in the reference orientation) that is between 20 degrees and 80 degrees, or e.g. between 30 degrees and 60 degrees, or e.g. about 45 degrees.

In some embodiments, the plane in which the maximum diameter of the proximal flange resides may be substantially parallel with the base, e.g. the surface of the base from which the stem extends or e.g. with a major surface of the base (e.g. with the base plane). The maximum diameter plane of the proximal flange may form an angle of e.g. 1 to 10 degrees with the base (e.g. the base plane).

In some embodiments, the plane in which the maximum diameter of the distal flange resides may be angled with respect to the surface of the base from which the stem extends or e.g. with the major surface of the base (and/or e.g. with the base plane). For example, the angle between the maximum diameter plane of the distal flange and the base (e.g. the base plane) may be between 20 degrees and 80 degrees, or e.g. between 30 degrees and 60 degrees, or e.g. about 45 degrees.

In some embodiments, the plane in which the maximum diameter of the proximal flange resides may be substantially perpendicular to the proximal portion of the elongate stem.

In some embodiments, the plane in which the maximum diameter of the distal flange resides may be substantially perpendicular to the distal portion of the elongate stem.

In some embodiments, the or each flange may each be non-planar. For example, each flange may define a dome-, cup- or cone-shape about the stem. In this way, each flange may define an annular volume within it.

Dome-, cup- or cone-shaped flanges (hereinafter referred to simply as "dome-shaped flanges") can advantageously provide an ergonomic fit within the human ear canal which further improves the overall comfort and performance of the earplug when in use.

The or each flange may continuously increase in diameter from a distal end to a proximal end thereof (i.e. may taper outwardly continuously in a direction towards the base). The proximal dome-shaped flange may continuously increase in diameter from a distal end to a proximal end thereof (i.e. may taper outwardly continuously in a direction towards the base). The distal dome-shaped flange may continuously increase in diameter from a distal end to a proximal end thereof (i.e. may taper outwardly continuously in a direction towards the base).

The or each flange may comprise a dome opening (i.e. an opening to an internal space enclosed by the dome). Each dome opening may comprise a perimeter (i.e. defining the opening in its interior). The proximal dome-shaped flange may comprise a proximal dome opening at the plane in which the maximum diameter of the proximal flange resides. The distal dome-shaped flange may comprise a distal dome opening at the plane in which the maximum diameter of the distal flange resides.

The proximal dome opening and distal dome opening each (generally) face towards the base of the earplug.

In some embodiments, the proximal flange may define a larger annular volume than the annular volume defined by the dome-shaped distal flange. In some embodiments, the proximal dome opening comprises a larger area than the area defined by the distal dome opening. The perimeter (e.g. circumference) of the proximal dome opening may be larger (i.e. longer in length) than the perimeter of the distal dome opening.

As the distal and proximal portions are angled, the planes of the dome openings may not be parallel (or co-planar) with each other. The plane of the opening for the proximal dome-shaped flange may be parallel to the base e.g. parallel to the major surface of the base. The plane of the opening for the distal dome-shaped flange may be at an angle to the base e.g. at an angle to the major surface of the base.

The plane in which the maximum diameter of the distal flange resides e.g. the plane of the distal dome opening may be orientated at an angle β to the plane in which the maximum diameter of the proximal flange resides and/or the plane of the proximal dome opening and/or the base plane (e.g. the plane of a major surface of the base) of the earplug.

In an example embodiment, the angle β may be any angle in the range between 5 and 85 degrees, such as between 10 and 80 degrees, for example between 15 and 75 degrees. The angle β may be any angle in the range between 20 and 70 degrees, such as between 25 and 65 degrees, for example between 30 and 60 degrees. In particularly preferred embodiments, the angle β may be any angle in the range between 35 and 55 degrees, such as between 40 and 50 degrees, for example between 42 and 48 degrees such as about 45 degrees.

As discussed above, each dome opening comprises a perimeter that defines a periphery of the flange. In one embodiment, the flange periphery/perimeter of the dome opening (e.g. of the distal flange) may define a symmetrical shape, such as a circle or oval. The stem (e.g. the distal portion) may transect the plane of the (e.g. distal) dome opening at or proximal the centre of the symmetrical shape e.g. at or proximal the centre of the (e.g. distal) dome opening. Where the (e.g. distal) dome opening is substantially circular, the dome-shaped distal flange may form a substantially hemispherical shape.

Symmetrical dome shapes may provide a good ergonomic fit at the narrow section of the ear canal. This may further advantageously provide a closer fit in the ear canal, thus further improving the comfort and performance of the earplug when worn.

In some embodiments, the flange periphery/perimeter of the (e.g. proximal) dome opening may be asymmetrically shaped, for example an asymmetrical ellipse. For example the periphery/perimeter may be asymmetrically shaped about an axis that extends through the part of the flange connected to the stem. The perimeter of the (e.g. proximal) dome opening may be generally D-shaped.

The perimeter of the (e.g. proximal) dome opening may have first and second sections, the first section having a smaller radius of curvature than the second section (i.e. the second section may be more linear/flatter than the first section). The second section may be on a forward side of the perimeter. The first section may be on a rearward side of the perimeter.

The perimeter of the (e.g. proximal or distal) dome opening may lie in a single plane (e.g. may not be scalloped or wavy).

Asymmetrical dome shapes may provide a good ergonomic fit at the wide section of the ear canal. This may advantageously provide a closer fit in the ear canal, thus improving the comfort and performance of the earplug when worn. The (e.g. proximal) dome may be formed so as to have an asymmetrical taper/slope (i.e. from a distal end of the flange to the perimeter of the opening). Thus, for example, a first side of the dome may have a shallower taper/slope than an opposing second side of the dome. The first side may be a rearward side (i.e. extending rearwardly in the reference orientation) and the second side may be a forward side (i.e. extending forwardly in the reference orientation).

In some embodiments, the stem and flanges may be formed of a flexible material. This flexible material may be, for example, a mouldable thermoplastic, thermoplastic rubber (TPR), or rubber. The flexible material allows the stem and flanges to conformably fit inside the ear canal of the wearer.

In some embodiments, the base comprises a connecting portion connected to the stem. The connecting portion may be integrally formed with the stem. Thus the connecting portion may be formed of the same material as the stem e.g. the connecting portion may be formed of TPR.

The connecting portion may be dish-shaped, defining a central recess. The central recess is provided on opposing surface to the stem.

The base may further comprise an insert seated within the connecting portion (e.g. within the central recess of the dish-shaped connecting portion) on an opposing surface of the connecting portion from which the stem extends. Together, the connecting portion and insert form the base with one major surface defined by the connecting portion (from which the stem extends) and the other major surface defined by the insert.

The upper edges of the dish-shaped connecting portion defining the central recess may be chamfered towards the insert.

The insert may be formed of the same material as the connecting portion (and stem) e.g. TPR.

TPR is waterproof material and has low-enough density that allows it to float in water, making it suitable for use in the manufacture of swimming earplugs. TPR is also recyclable material so, by forming all of the components of the earplugs with TPR, the earplug may be broken down and fully recycled at the end of product's life. Thus, the environmental sustainability of the entire earplug product is improved.

In an exemplary embodiment, the insert and connecting portion of the base, the stem, and the flanges disposed on the stem are all formed of TPR. In this way, the earplug product is a fully recyclable product. In some embodiments, a fixed connection is formed between the connecting portion and insert by over-moulding the connecting portion onto the insert during a manufacturing process of the earplug. This over-moulding fixedly connects or bonds the connecting portion and insert together.

In other embodiments, the base may be a single piece (i.e. may not be formed by a connecting portion and an insert). In such embodiments the base may be formed of TPR and may be integral with the stem and flanges. Thus, the earplug may be unitary structure.

In some embodiments, the base (e.g. the insert) may further comprise a handle for the wearer to grip on to during use of the earplug. The handle may be integrally formed with the base (e.g. insert) and formed of the same material e.g. TPR. The handle may be elongate and may extend from the base of the earplug e.g. in a substantially downwards direction (when in the reference orientation). For example, in the reference orientation the handle may extend in a downward direction that forms an (inner) angle with vertical of between 0 degrees and 45 degrees, or between 5 degrees and 30 degrees.

In a preferred embodiment, the elongate handle extends away from the base of the earplug in a direction perpendicular, or substantially perpendicular, to the proximal portion of the stem. When the earplug is fully inserted in the ear canal, the elongate handle advantageously provides a lever by which the wearer can easily leverage the earplug out of the ear canal with minimum effort. For example, when the earplug is in use and is in situ in the ear canal, the wearer can lift the handle away from the wearer's face and dislodge the earplug from the ear canal.

In some embodiments, a distal portion of the handle (distal from the base) may be angled with respect to a proximate portion of the handle (proximate to the base). The distal portion may be angled in a direction towards user's head when worn (i.e. inwardly in the reference orientation). Such shape may allow the handle to hug the face of a wearer when worn. This may be particularly beneficial, for example, when the earplugs are used for swimming, whereby the force applied by the water (through which a wearer is moving) on the handle could otherwise dislodge the earplugs from a wearer's ear.

In some embodiments, the elongate handle may be curved along its length. The curvature of the handle may allow it to curve around (i.e. clear) the external part of the ear when received in a user's ear canal. The curvature of the handle may be such that it curves (inwardly) towards a user's head when worn. The handle may include insignia such as a logo or symbol. For example, the handle may comprise a letter indicating to wearer whether the earplug belongs to the left ear (e.g. insignia reciting the letter "L") or the right ear (e.g. insignia reciting the letter "R").

The connecting portion may comprise a cut-out for the handle to extend through.

The stem and flanges may be softer and/or more flexible than at least a portion of the base. For example, the stem and flanges may be softer than the insert when present.

The insert (when present) may have an increased hardness relative to the connecting portion and/or stem/flanges e.g. the insert may be formed of harder TPR than the TPR used to form the connecting portion/stem/flanges. The base may have an increased hardness relative to the stem and/or flanges (e.g. may be formed of a harder TPR).

The harder base (e.g. insert) may be more easily handled or gripped by the wearer. It advantageously aids the wearer in handling, gripping, and inserting the earplug into the ear canal.

The softer connecting portion/stem/flanges provides for increased comfort within the user's ear.

Hardness is typically quantified using a 'Shore A Hardness' (SHA) value.

In an example embodiment, the stem, the flanges, and/or the connecting portion of the base may be formed of a material having any SHA value in the range between 20 and 80 SHA, such as between 25 and 75 SHA, for example between 30 and 70 SHA. In some embodiments, the stem, the flanges, and/or the connecting portion of the base may be formed of a material having any SHA value in the range between 35 and 65 SHA, such as between 40 and 60 SHA, for example between 25 and 55 SHA such as about 50 SHA.

In another example embodiment, the handle and/or the insert of the base may be formed of a material having any SHA value in the range between 115 and 80 SHA such as between 110 and 85 SHA, for example between 105 and 90 SHA. In some embodiments, these components may be formed of a material having any SHA value in the range between 100 and 90 SHA, such as about 95 SHA.

In some embodiments, the base may comprise a connector (e.g. a male connector or a female connector) for interconnection with another earplug. The base may, for example, comprise a recess or a protrusion. The connector may be formed on a surface of the base opposing the stem.

The connector may be as otherwise described below with respect to the second aspect.

In a second aspect, there is provided a pair of earplugs comprising:
a first earplug comprising a first connector, and
a second earplug comprising a second connector,
wherein the first connector of the first earplug is releasably engageable with the second connector of the second earplug.

These connectors are particularly useful when storing a pair of the earplugs together when they are not in use (e.g. when packing them together away into a sports bag). This advantageously allows both earplugs to be held together via the connectors when they are not in use so that they are less likely to become lost or displaced from one another during transit.

The first connector may be a male connector. The second connector may be a female connector.

The male connector may be configured to be releasably received within the female connector e.g. to form an interference fit therebetween e.g. a snap-fit connection.

The first and/or second earplug may be as described for the first aspect.

For example, the or each ear plug may comprise a base (e.g. as described for the first aspect) and an elongate stem extending from the base. The first/second connector may be provided on the base (e.g. on an opposite side of the base to the stem). The or each ear plug may comprise a stem as described for the first aspect and the first/second connector may be provided on the base on the opposing major surface to that from which the stem extends.

The stem may comprise flanges as described for the first aspect.

The base may comprise an insert and connecting portion as described for the first aspect and the first/second connectors may be provided on the insert.

The female connector may be a recess e.g. a substantially circular recess. The male connector may be projection or boss shaped and dimensioned to be received in the recess. As discussed above, the recess and protrusion may be formed on the base e.g. on the insert of the respective earplug.

In some embodiments, the recess may have a depth ranging between 0.25mm to 2.00mm. In an example embodiment, the recess may have a depth ranging between 0.50mm and 1.75mm, such as between 0.75mm and 1.50mm. In some embodiments, the recess may have a depth ranging between 0.75mm and 1.25mm, for example having an internal diameter of about 1.00mm.

In some embodiments, the recess may have an internal diameter ranging between 6.0mm and 10.0mm, for example between 6.5mm and 9.5mm, such as between 7.0mm and 9.0mm, for example having internal diameter of between 7.5 and 8.5mm e.g. about 8.0mm.

The projection of the male connector e.g. the boss may have an extension matching that of the recess i.e. within the ranges given above for the depth of the recess.

The external diameter of the male connector e.g. the boss may match the internal diameter of the recess i.e. within the ranges given above for the internal diameter of the recess.

In use, the interconnection of the earplugs may be performed by hand by the wearer. To illustrate the process, the male connector (e.g. boss) of the first earplug is brought into contact with, and pushed into, the female connector (e.g. recess) of the second earplug by the wearer. The force required to push the connectors together is exerted by hand in order to form an interference fit connection therebetween. This interference fit is a detachable connection, such as a 'snap-fit' connection. The force required to detach the connectors from one another may also be exerted by hand, such the detachment is also manually performed by the wearer.

In some embodiments, the male/female connectors may include insignia such as an embossed logo or symbol. They may comprise a letter indicating whether the earplug belongs to the left ear (e.g. a letter "L") or the right ear (e.g. a letter "R"). These letters help the wearer identify whether the earplug is meant for insertion in the left or right ear.

### Brief Description of the Drawings

Figure 1 is a schematic anatomical view of a human ear canal;
Figure 2A is a top view of an earplug;
Figure 2B is a wireframe view of the earplug shown in Figure 2A;
Figure 3 is an exploded view of the ear plug of Figure 2A;
Figures 4 is a rear perspective view of the earplug of Figure 2A;
Figure 5 is a perspective of view of a pair of earplugs;
Figure 6A is a side of view of the first earplug shown in Figure 5;
Figure 6B is a side view of the second earplug shown in Figure 5;
Figure 7A is a perspective view of the first and second earplugs when interconnected.
Figure 7B is a detailed perspective view of the first and second earplugs of Figure 7A.

### Detailed Description and Further Optional Features

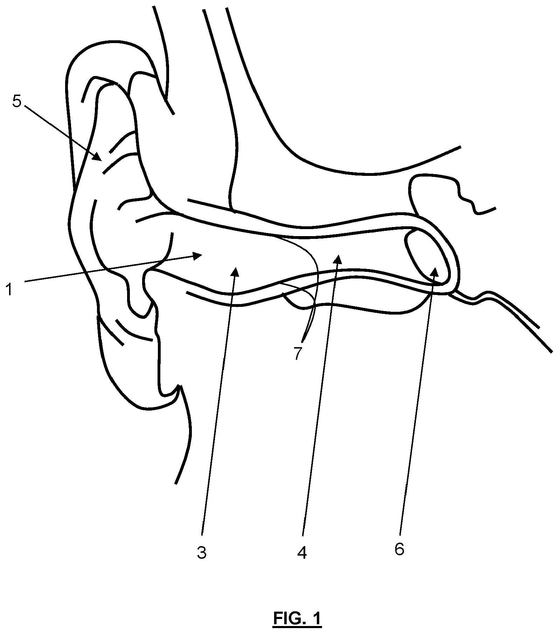

Figure 1 shows a schematic anatomical view of a human ear canal 1. The human ear canal 1 is a pathway, formed by a hollow cavity in the temporal bone of a human skull, and runs from an outer ear 5 (i.e. the external part of the ear) up to the eardrum 6 inside the ear canal 1.

Referring to Figure 1, the ear canal has a shape that (moving inwardly towards the eardrum 6) begins with a wide section 3, where the internal walls 7 of the ear canal 1 are farthest apart and is located near to the outer ear 5. Moving towards the ear drum 6 from the wide section 3, the ear canal 1 tapers inwardly in an upward direction to form a narrow section 4 of the ear canal 1, where the internal walls 7 are closer, and is located deeper inside of the ear canal 1. As the name suggests, the wide section 3 of the ear canal 1 refers to a section of the ear canal 1 that is wider than the narrow section 4 inside of the ear canal 1.

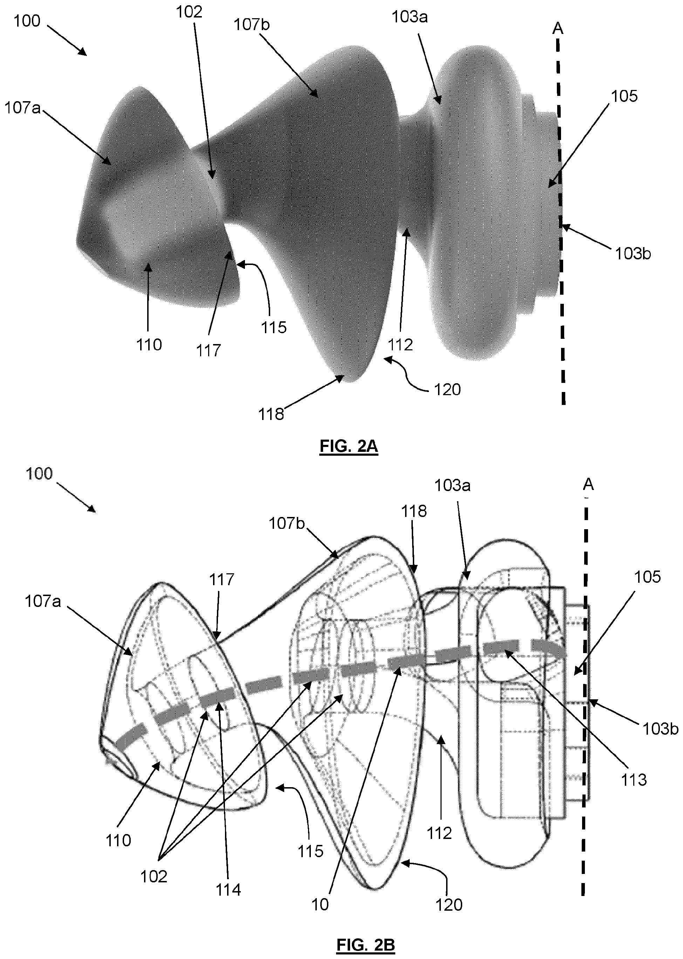

Figure 2A is a top view of an earplug 100 (i.e. viewed from above when in the reference orientation). The earplug 100 includes an elongate stem 102 (partially visible in this figure), which has a proximal portion 112 proximate to a base 105, and a distal portion 110 distal to the base 105. As apparent from the figure, the distal portion 110 extends at an angle to the proximal portion 112. The proximal portion 112 is connected to the base 105. The base 105 is the part of the earplug 100 that remains external to, or outside of, the ear canal 1 when the earplug 100 is being worn, whereas the stem 102 is the part of the earplug 100 that fits inside the ear canal 1 of the wearer when the earplug 100 is being worn.

Figure 2B is a wireframe view of the earplug shown in Figure 2A. As is apparent from this figure, the elongate stem 102 is curved along a portion of its length (indicated by the curved dashed line 10). In particular, in the embodiment shown, the distal portion 110 of the stem 102 is curved. The base includes two major surfaces: a first major surface 103a from which the stem 102 extends and a second major surface 103b opposite the first major surface 103a. The stem 102 extends within a plane that is substantially perpendicular to the major surfaces 103a, 103b of the base 105. Accordingly, the curvature of the distal portion 110 is also within a plane that is substantially perpendicular to the major surfaces 103a, 103b of the base 105.

The proximal portion 112 of the stem 102 has a transverse cross-section (perpendicular to the elongation of the stem 102) that decreases in a direction away from the base 105. In this way, the stem 102 has a maximum cross-sectional area adjacent to the base 105, so to as to provide a secure attachment to the base 105.

There are two flanges disposed on the stem 102: a proximal flange 107b and a distal flange 107a. The proximal flange 107b is disposed on (and integrally formed with) the proximal portion of the stem 102. The distal flange 107a is disposed on (and integrally formed with) the distal portion of the stem 102. Thus, the flanges 107a, 107b are integrally formed with the stem 102 and are formed of the same material as the stem 102 (i.e. such that the flanges 107a, 107b and stem 102 form a unitary body).

As the distal flange 107a is disposed at an end of the elongate stem 102 distal to the base 105, in use, the distal flange 107a abuts against the internal walls 7 at the narrow section 4 of the ear canal 1. On the other hand, the proximal flange 107b (which is proximate to the base 105) abuts against internal walls 7 at the wide section 3 of the ear canal 1. By providing two flanges spaced along the stem 102 in this way, the two flanges 107a, 107b more closely correspond to the internal shape 2 of the ear canal 1 at its widest and narrowest sections. This improves the overall comfort and performance of the earplug 100 in use.

Each flange 107a, 107b has a maximum diameter defined by a maximum dimension of that flange 107a, 107b in a radial direction. The proximal flange 107b is larger than the distal flange 107a and, in particular, the proximal flange 107b has a larger maximum diameter than the maximum diameter of the distal flange 107a. In other words, a perimeter 118 of the proximal flange 107b has a greater diameter than a perimeter of the 117 of the distal flange 107a.

This allows the distal flange 107a to abut against the internal walls 7 of the user's ear canal 1 at its narrower (deeper) section 4, whereas the larger proximal flange 107b can abut against the internal walls 7 of the ear canal 1 at the wider (shallower) section 4. In this way, the maximum diameters of the two flanges respectively match the ear canal 1 at its widest and narrowest sections. This improves the overall comfort and performance of the earplug 100 when in use.

As a result of the curvature of the curved elongate stem 102, the planes in which the maximum diameters of the flanges reside are not parallel with one another. Likewise, neither plane is parallel to the base 105 (i.e. neither plane is parallel to the major surfaces 103a, 103b of the base 105).

The base 105 of the earplug 100 defines a reference place, referred to herein as "base plane" and indicated by dashed line A in figures 2A and 2B. As should be apparent, the base plane A is parallel to both of the major surfaces 103a, 103b of the base 105. The elongate stem 102 extends away from the base 105 and includes a linear portion 113 and a curved portion 114. The linear potion 113 is proximal to the base 105 and the curved portion 114 is distal to the base 105. The linear portion 113 of the elongate stem 102 is perpendicular to the base plane A.

The proximal flange 107b and the distal flange 107a are dome-shaped. Each flange 107a, 107b is rounded or curved to form a dome, such that a tapered annular volume (apparent from Figure 2B) is defined between each flange 107a, 107b and the stem 102. The dome-shaped flanges 107a, 107b provide an ergonomic fit within the human ear canal which further improves the overall comfort and performance of the earplug when in use.

The proximal dome-shaped flange 107b comprises a proximal dome opening 120 at the plane in which the maximum diameter 118 of the proximal flange 107b resides. Likewise, the distal dome-shaped flange 107a comprises a distal dome 115 opening at the plane in which the maximum diameter 117 of the distal flange 107a resides. The proximal dome opening 120 of the proximal dome-shaped flange 107b and distal dome opening 118 of the distal dome-shaped flange 107a each face towards the base 105 of the earplug 100.

As the elongate stem 102 is curved along its at least a portion of its length, the planes in which dome openings 118,120 reside are not parallel with each other. The plane of the opening 120 of the proximal dome-shaped flange 107b is parallel to the plane of the base A. The plane of the opening 118 for the distal dome-shaped flange 107a is at an angle to the plane of the base A (i.e. is obliquely arranged with respect to the plane of the base A).

Each opening 115, 120 of the dome-shaped flanges 107a, 107b is defined within a perimeter 117, 118 of the respective flange 107a, 107b. The perimeter 118 of the proximal flange 107b has an elliptical shape that is asymmetrical about the elongate stem 102. In this way, the proximal flange 107b forms an asymmetrical dome shape. The asymmetrical perimeter 118, and the fact that the proximal portion 112 doesn't extend centrally through the proximal flange 107b, means that the proximal flange 107b has an asymmetrical taper. In particular, a rearward side (extending downward on the page) has a shallower slope than a forward side (extending upward on the page).

Asymmetrical dome shapes can provide a better ergonomic fit at the wide section 3 of the ear canal 1. This may advantageously provide a closer fit within the ear canal 1, thus improving the comfort and performance of the earplug 100 when worn.

The perimeter 117 of the distal flange 107a has a circular shape that is symmetrical about the elongate stem 102. Likewise, the distal flange 107a itself forms a symmetrical (or hemispherical) shape. Symmetrical dome shapes can provide a better ergonomic fit at the narrow section 4 of the ear canal 1. This may further advantageously provide a closer fit within the ear canal 1, thus further improving the comfort and performance of the earplug 100 when worn.

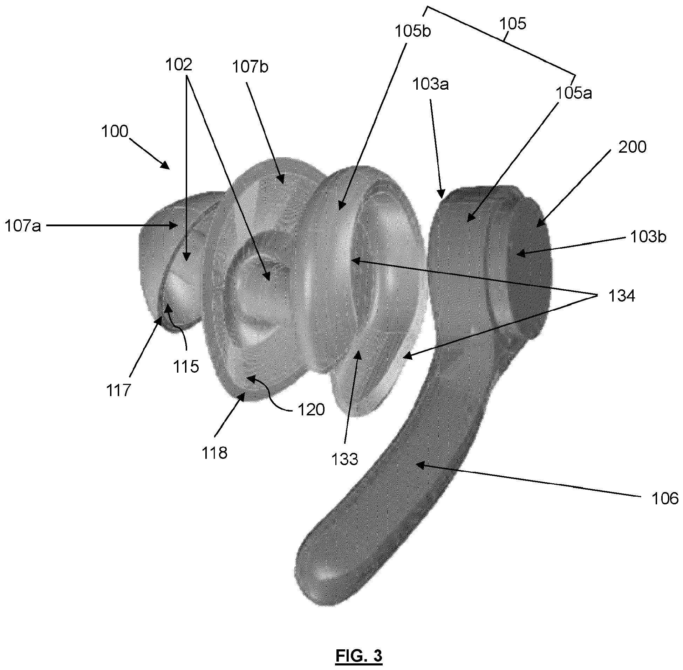

Figure 3 is an exploded view of the ear plug 100. It is once again apparent from this figure that the proximal flange 107b is larger than the distal flange 107a. Likewise, the perimeter 118 of the opening 120 of the proximal flange 107b is larger than the perimeter 117 of the opening 115 of the distal flange 107a. By having a larger perimeter 118, the opening 120 of the proximal flange 107b defines a larger annular volume than the annular volume defined by the opening 115 of the distal flange 107a.

The base 105, the elongate stem 102, and the flanges 107a and 107b are formed of thermoplastic rubber (TPR). TPR is a waterproof material and has low-enough density that allows it to float in water, making it suitable for use in the manufacture of swimming earplugs. TPR is also a recyclable material so, by forming all of the components of the earplugs with TPR, the earplug may be broken down and fully recycled at the end of product's life. Thus, the environmental sustainability of the entire earplug product is improved.

The stem 102 and the flanges 107a, 107b are formed of a softer TPR than the TPR used to form the base 105. In this embodiment, the stem 102 and the flanges 107a, 107b are formed of TPR having a SHA value of 50 SHA and the base 105 is formed of TPR having a SHA value of 95 SHA.

Also apparent from Figure 3, is that the base 105 comprises a connecting portion 105b connected to the stem 102. The connecting portion 105b is integrally formed with the stem 102, such that, like the stem 102, the connecting portion 105b is formed of TPR.

The connecting portion 105b is dish-shaped and defines a central recess 133. The central recess 133 is provided on an opposing surface to that from which the stem 102 extends. The base 105 further comprises an insert 105a seated within the connecting portion 105b (i.e. within the central recess 133 of the dish-shaped connecting portion 105b). In other embodiments, the stem may extend directly from the insert 105a (i.e. the connecting portion 105b may be omitted).

During the manufacturing process of the earplug 100, the connecting portion 105b is over-moulded onto the insert 105a to form a fixed connection between the connecting portion 105a and the insert 105b. This forms the base 105 of the earplug 105, with one major surface 103a defined by the connecting portion 105b (from which the stem 102 extends) and the other major surface 103b defined by the insert 105a. Upper edges 134 of the dish-shaped connecting portion 105b defining the central recess 133 are chamfered towards the insert 105a.

The insert 105a has a handle 106 for gripping by the wearer. The handle 106 is integral with the remainder of the insert 105a and is thus formed of TPR. In this way, the insert 105a, connecting portion 105b, handle 106, elongate stem 102, and flanges 107a, 107b are all formed of TPR. Accordingly, the earplug product is a fully recyclable product.

The connecting portion is formed of a softer TPR than the TPR used to form the insert 105a and the handle 106. In the embodiment shown, the connecting portion 105b, the stem 102, and the flanges 107a, 107b are formed of TPR having a SHA value of 50 SHA. The insert 105a and the handle 106 are formed of TPR having a SHA value of 95 SHA. The softer TPR of the flanges 107a, 107b and the stem 102 allows them to be flexible enough to mould to the shape of the ear canal 1 and provide a comfortable and snug fit when fully inserted into the ear canal 1 of a wearer. In contrast, the hardness of the handle 106 allows it to be handled or gripped more easily by a wearer. That is, it advantageously aids the wearer in handling, gripping, and inserting the earplug into the ear canal 1.

The insert 105a of the base 105 has a connection mechanism 200 for interconnection with a further earplug (not shown in Figure 3). The connection mechanism 200 is located on an opposite side of the base 105 to the stem 102 and projects in a direction facing away from the ear canal 1 when in use. In this way, the connection mechanism 200 allows one earplug 100 to be fixedly connected, or attached to, the further earplug (not shown). The connection mechanism 200, in the illustrated embodiment, is a male connector for connection to a female connector (not shown) of the further earplug.

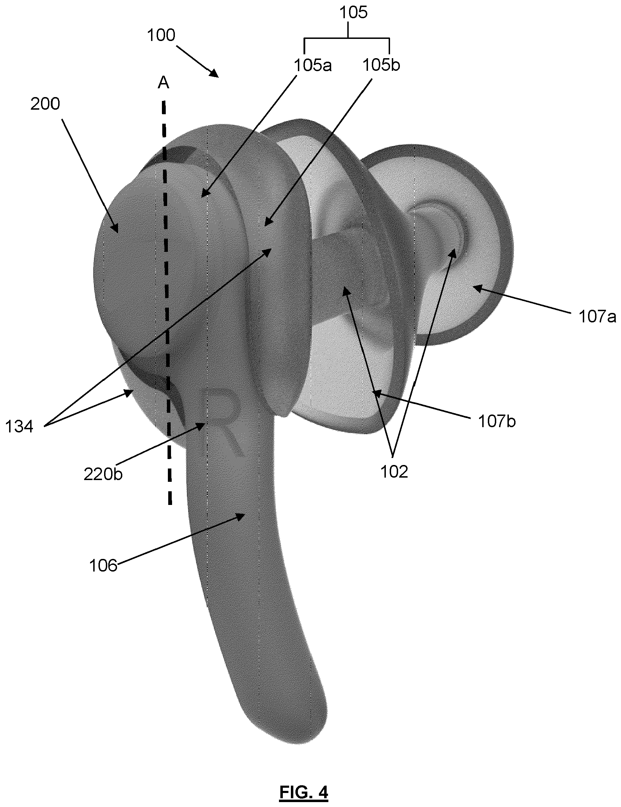

Figure 4 is a rear perspective view of the earplug 200 shown in Figure 3 and provides a further view of the handle 106. The handle 106 is elongate and extends away from the base 105 in a direction substantially parallel to the plane of the base A (as illustrated by dashed line A in Figure 4). When the earplug 100 is fully inserted in the ear canal 1, the elongate handle 106 advantageously provides a lever by which the wearer is able to leverage the earplug 100 out of the ear canal 1. In one embodiment, when the earplug 100 is in situ in the ear canal 1 (not shown in this figure), the wearer lifts the handle 106 away from the wearer's face and dislodges the earplug 100 from the ear canal 1.

The elongate handle 106 is curved along its length towards the stem 102 of the earplug 100. The curvature of the handle 106 provides a gripping feature for ease-of-handling by the wearer, allowing the curved handle 106 to be easily gripped between the thumb and index finger of the wearer. The handle 106 has insignia 200b reciting the letter "R" indicating to the wearer that the earplug 100 belongs to the right ear.

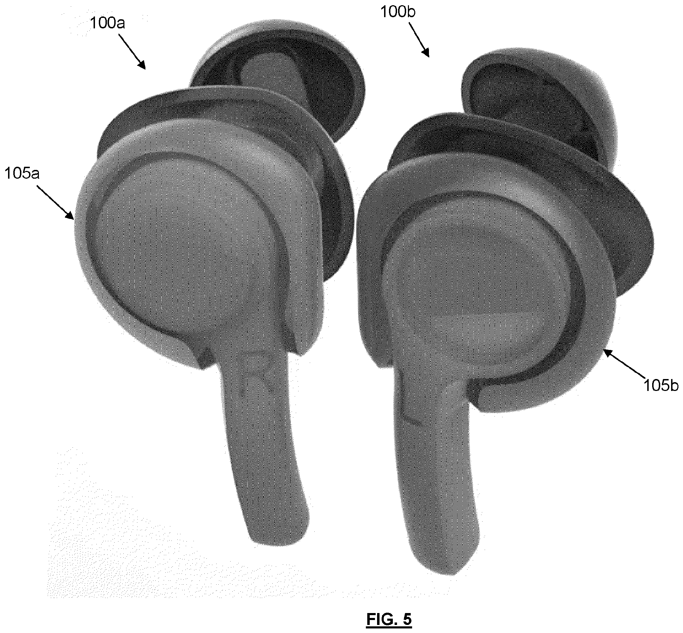

Figure 5 is a perspective view a pair of first 100a and second 100b earplugs. The first earplug 100a is the same as that described above and illustrated in Figures 2A to 4.

The first earplug 100a is dimensioned to fit inside the ear canal 1 of right ear, and the second earplug 100b is dimensioned to fit inside the ear canal 1 of the left ear of the wearer. In this way, the first and second earplugs 100a, 100b are mirror-image duplications of each other (i.e. identical in form but reversed).

The first earplug 100a has a male connector 200a on its base 105a, and the second earplug 100b has a female connector 200b on its base 105b. The male connector 200a of the first earplug 100a is correspondingly dimensioned to engage with the female connector 200b of the second earplug 100b to form an interference fit therebetween. In particular, the male connector 200a of the first earplug 100a is boss and the female connector 200b of the second earplug 100b is a correspondingly dimensioned recess for receiving the boss of the first earplug 100a.

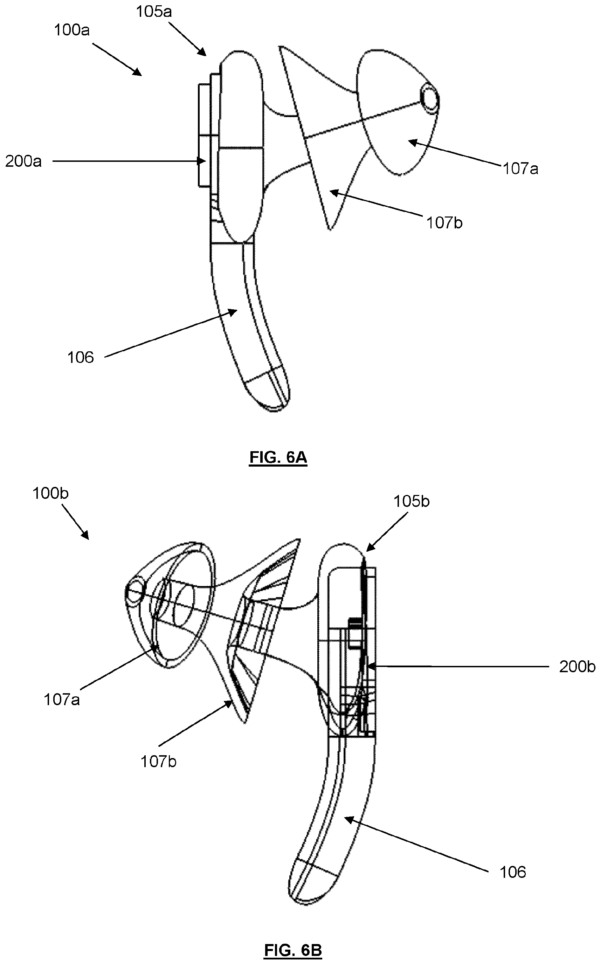

Figures 6A and 6B more clearly show the male 200a and female 200b connectors. Figure 6A is a side of view of the first earplug 100a shown in Figure 5. As is apparent from this figure, the male connector 200a is a circular boss with a length of 1 mm and an external diameter of 8mm. Figure 6B is a side view of the second earplug 100b shown in Figure 5. As is apparent from this figure, the female connector 200b is a circular recess with a depth of 1 mm and an internal diameter of 8mm.

In use, the interconnection of the earplugs 100a, 100b can be performed by hand by the wearer. The boss 200a of the first earplug 100a is brought into contact with, and pushed into, the recess 200b of the second earplug by the wearer. The force required to push the boss 200a into the recess 200b is exerted by hand and this results in an interference fit connection between the earplugs 100a, 100b. This interference fit is a detachable connection. The force required to detach the boss 200a from the recess 200b is also exerted by hand, such the detachment is also manually performed by the wearer.

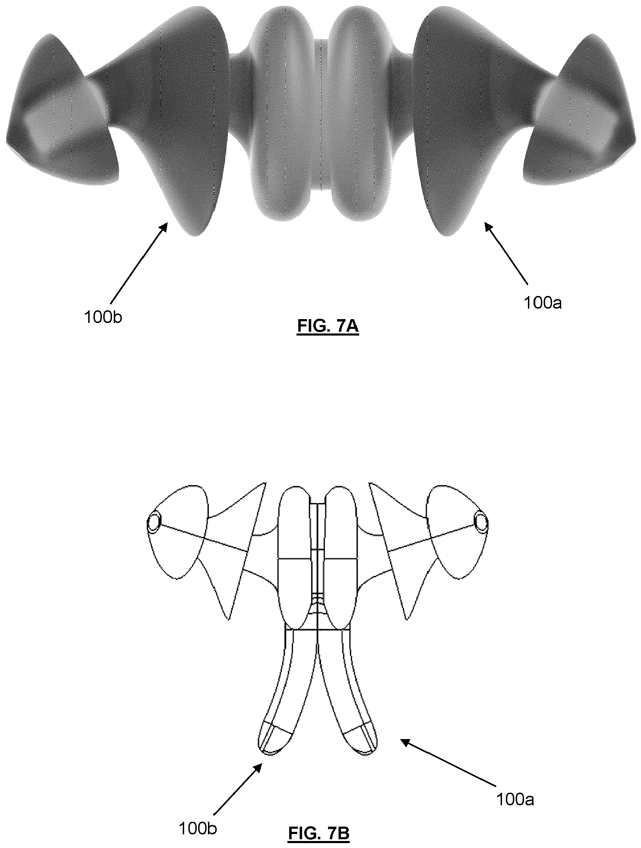

The earplugs 100a, 100b are illustrated in there attached configuration in figures 7A and 7B. As illustrated in these figures, the earplugs 100a, 100b are retained in this interconnected configuration once the connection has been made. This is particularly useful when storing a pair of the earplugs together when they are not in use (e.g. when packing them together away into a sports bag). That is, the earplugs 100a, 100b can be held together via the connection mechanism when they are not in use so that they are less likely to become lost or displaced from one another during transit.

## Claims

1. An earplug comprising:
a base;
an elongate stem extending from the base for insertion into a user's ear canal, the stem comprising a proximal portion proximate to the base and a distal portion distal from the base, the distal portion extending at an angle to the proximal portion; and
one or more flanges provided on the stem.

2. An earplug according to claim 1 wherein the stem is curved along at least a portion of its length.

3. An earplug according to claim 1 or 2 wherein the proximal portion extends from the base at an oblique angle.

4. An earplug according to claim 3 wherein the proximal portion extends from the base in an inward and upward in-use direction.

5. An earplug according to claim 3 or 4 wherein the proximal portion extends from the base in an inward and rearward in-use direction.

6. An earplug according to any one of the preceding claims wherein the distal portion extends in an inward and rearward in-use direction from the proximal portion.

7. An earplug according to any one of the preceding claims wherein the one or more flanges comprises a distal flange disposed on the distal portion and a proximal flange disposed on the proximal portion, the proximal flange having a larger maximum diameter than the distal flange, and optionally a maximum diameter plane of the proximal flange forms an angle of between 20 and 80 degrees with the maximum diameter plane of the distal flange.

8. An earplug according to claim 7 wherein the maximum diameter plane of the proximal flange is substantially parallel to the base.

9. An earplug according to claim 7 or 8 wherein the proximal flange has a larger maximum diameter than the distal flange.

10. An earplug according to any one of the preceding claims wherein the periphery of one of the one or more flanges is asymmetrical

11. An earplug according to claim 10 wherein the asymmetrical flange is dome shaped and a first side of the dome has a shallower slope than an opposing second side of the dome.

12. An earplug according to any one of the preceding comprising an elongate handle extending from the base.

13. An earplug according to claim 12 wherein the handle is curved along its length.

14. A pair of earplugs comprising:
a first earplug comprising a first connector, and
a second earplug comprising a second connector,
wherein the first connector of the first earplug is releasably engageable with the second connector of the second earplug.

15. A pair of earplugs according to claim 14 wherein the first connector is a male connector and the second connector is a female connector, and optionally wherein the first connector comprises a protrusion and the second connector comprises a recess, and further optionally wherein each earplug comprises a base and a stem extending from the base, the protrusion provided on the base of the first earplug and the recess provided on the base of the second earplug.
